# EUROPEAN PATENT APPLICATION

(11) **EP 4 129 381 A1**
(43) Date of publication of application: **08.02.2023**
(21) Application number: 21780029.1
(22) Date of filing: 31.03.2021
(51) Int. Cl.: A61M 25/00, A61B 18/04, A61B 18/24

(54) **BALLOON-EQUIPPED CATHETER**

(30) Priority: 31.03.2020 JP 2020064360
(71) Applicant: Toray Industries, Inc., Tokyo 103-8666 (JP)
(72) Inventor: HARADA Hiroyuki, Tokyo 103-8666 (JP); OKA Yumi, Otsu-shi, Shiga 520-2141 (JP); FUJII Kouhei, Otsu-shi, Shiga 520-8558 (JP)
(74) Representative: Kador & Partner PartG mbB
(86) International application number: PCT/JP2021/013778
(87) International publication number: WO 2021/201076

(57) **Abstract**

A balloon catheter (10) comprises: a housing (55); a catheter shaft (20) having a proximal end portion disposed in the housing (55) and forming a liquid feeding path (21) communicating with an inside of a balloon (15) attached to a distal end portion; a supply-discharge line connection portion (30) which has an internal space (31) communicating with the liquid feeding path (21), and to which a supply-discharge line (36) that supplies and discharges liquid to and from the liquid feeding path (21) through the internal space (31) can be connected; and a stopcock (35) capable of closing the internal space (31) of the supply-discharge line connection portion (30). A position of the supply-discharge line connection portion with respect to the housing is fixed.

## Description

### TECHNICAL FIELD

The present invention relates to a balloon catheter.

### BACKGROUND ART

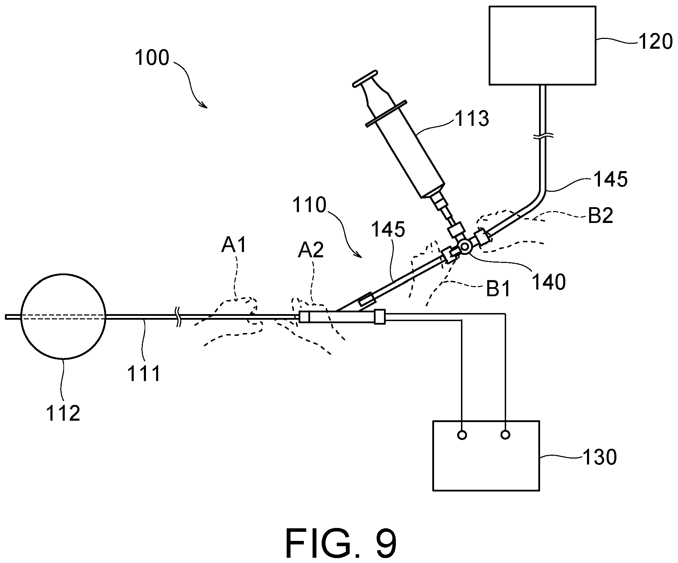

As shown in JP 5913739 B2, in recent years, treatment using a balloon catheter has been performed. Balloon catheters are used to cauterize myocardial tissue with heated liquid in the balloon, for example, in the treatment of arrhythmias. FIG. 9 illustrates a catheter system 100 to which a conventional balloon catheter 110 is applied.

The catheter system 100 illustrated in FIG. 9 includes a balloon catheter 110, a stirring device 120 that stirs liquid in the balloon catheter 110, and a heating device 130 that heats the liquid. The balloon catheter 110 includes a catheter shaft 111 in which a liquid feeding path is formed, a balloon 112 attached to a distal end portion of the catheter shaft 111, and a syringe 113 that supplies and discharges liquid to and from the liquid feeding path of the catheter shaft 111 and the balloon 112. The syringe 113 and the stirring device 120 are connected to the catheter shaft 111 via a three-way stopcock 140 and a flexible tube 145. By operating the three-way stopcock 140, one of the syringe 113 and the stirring device 120 can communicate with the liquid feeding path of the catheter shaft 111.

Such an operation of the balloon catheter 110 is very complicated because it is necessary to operate the three-way stopcock 140 and the like while holding the catheter shaft 111 inserted into the body of the patient. Therefore, the operation of the balloon catheter 110 requires a plurality of personnel. For example, as indicated by broken lines in FIG. 9, while a person holds the catheter shaft 111 with his/her both hands A1 and A2, another person operates the three-way stopcock 140 or the like with his/her both hands B1 and B2.

Meanwhile, it is required to improve the operability of the balloon catheter so that the number of personnel required for operating the balloon catheter can be reduced.

### DISCLOSURE OF THE INVENTION

The present invention has been made in view of the above points, and an object thereof is to provide a balloon catheter with improved operability.

A balloon catheter according to the present invention comprises: a housing; a catheter shaft having a proximal end portion disposed in the housing and forming a liquid feeding path communicating with an inside of a balloon attached to a distal end portion of the catheter shaft; a supply-discharge line connection portion which has an internal space communicating with the liquid feeding path, and to which a supply-discharge line that supplies and discharges liquid to and from the liquid feeding path through the internal space can be connected; and a stopcock capable of closing the internal space of the supply-discharge line connection portion, in which a position of the supply-discharge line connection portion with respect to the housing is fixed.

The balloon catheter may further comprise: the supply-discharge line connected to the supply-discharge line connection portion; and a supply-discharge device that is connected to the supply-discharge line to supply and discharge liquid to and from the supply-discharge line, and the supply-discharge line may include a soft tube having a length of 200 mm or more.

The balloon catheter may further comprise a stirring line connection portion which has an internal space communicating with the liquid feeding path, and to which a stirring line that sucks and discharges liquid from and into the liquid feeding path through the internal space of the stirring line connection portion to stir liquid in the balloon can be connected, and a connection portion between the internal space of the stirring line connection portion and the liquid feeding path may be located closer to the distal end portion of the catheter shaft than a connection portion between the internal space of the supply-discharge line connection portion and the liquid feeding path.

In the balloon catheter, the stirring line connection portion may be fixed to the housing.

In the balloon catheter, the catheter shaft may include an inner cylinder shaft to which a distal end of the balloon is fixed and an outer cylinder shaft to which a proximal end of the balloon is fixed, the outer cylinder shaft having a lumen into which the inner cylinder shaft is inserted to form the liquid feeding path between the outer cylinder shaft and the inner cylinder shaft, and the housing may be provided with an operation unit that extends the balloon by moving the inner cylinder shaft relative to the outer cylinder shaft along a first direction in which the proximal end portion of the catheter shaft extends.

In the balloon catheter, the housing may include a narrow portion narrowed in a direction intersecting a first direction in which the proximal end portion of the catheter shaft extends, a first gripping portion connected to the narrow portion from one side in the first direction, and a second gripping portion connected to the narrow portion from the other side in the first direction.

In the balloon catheter, the catheter shaft may include an inner cylinder shaft to which a distal end of the balloon is fixed and an outer cylinder shaft to which a proximal end of the balloon is fixed, the outer cylinder shaft having a lumen into which the inner cylinder shaft is inserted to form the liquid feeding path between the outer cylinder shaft and the inner cylinder shaft, a maximum outer radius of the first gripping portion about an axis extending along the first direction may be larger than a maximum outer radius of the second gripping portion about an axis extending along the first direction, and the first gripping portion may be provided with an operation unit that moves the inner cylinder shaft relative to the outer cylinder shaft along the first direction to extend the balloon.

Alternatively, a balloon catheter according to the present invention comprises: a housing; and a catheter shaft having a proximal end portion disposed in the housing and forming a liquid feeding path communicating with an inside of a balloon attached to a distal end portion, in which the housing includes a narrow portion narrowed in a direction intersecting a first direction in which the proximal end portion of the catheter shaft extends, a first gripping portion connected to the narrow portion from one side in the first direction, and a second gripping portion connected to the narrow portion from the other side in the first direction.

Alternatively, a balloon catheter according to the present invention comprises: a housing;
a catheter shaft having a proximal end portion disposed in the housing and forming a liquid feeding path communicating with an inside of a balloon attached to a distal end portion of the catheter shaft;
a supply-discharge line connection portion which has an internal space communicating with the liquid feeding path, and to which a supply-discharge line that supplies and discharges liquid to and from the liquid feeding path through the internal space of the supply-discharge line connection portion can be connected; and
a stirring line connection portion which has an internal space communicating with the liquid feeding path, and to which a stirring line that sucks and discharges liquid from and into the liquid feeding path through the internal space of the stirring line connection portion to stir liquid in the balloon can be connected,
in which a connection portion between the internal space of the stirring line connection portion and the liquid feeding path is located closer to the distal end portion of the catheter shaft than a connection portion between the internal space of the supply-discharge line connection portion and the liquid feeding path.

According to the present invention, it is possible to provide a balloon catheter with improved operability.

### BRIEF DESCRIPTION OF DRAWINGS

FIG. 1 is a view for explaining an embodiment of the present invention, and is a view illustrating an overall configuration of a catheter system to which a balloon catheter is applied.
FIG. 2 is a side view illustrating a proximal end portion of the balloon catheter illustrated in FIG. 1.
FIG. 3 is a cross-sectional view schematically illustrating a catheter shaft and a balloon.
FIG. 4 is a cross-sectional view schematically illustrating the catheter shaft and the balloon.
FIG. 5 is a diagram illustrating a method of operating an operation unit.
FIG. 6 is a view illustrating a partial cross-sectional view of a housing taken along line I-I of FIG. 1 together with a movement restricting protrusion of an operation unit.
FIG. 7 is a view illustrating a method of operating a stopcock.
FIG. 8 is a front view of the housing as viewed from a distal end side of the housing.
FIG. 9 is a diagram illustrating an overall configuration of a conventional catheter system.

### MODE FOR CARRYING OUT THE INVENTION

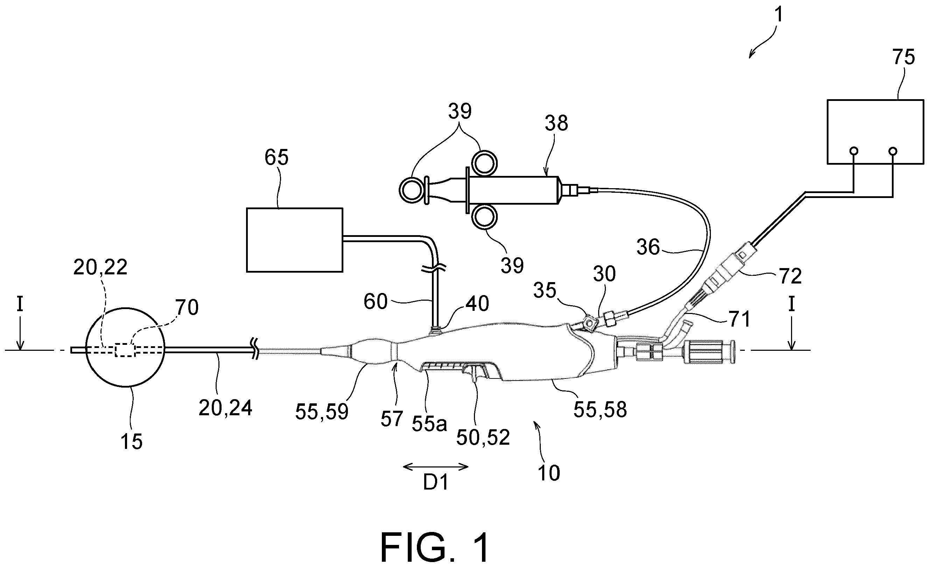

FIG. 1 is a view for explaining a balloon catheter according to the present embodiment. FIG. 1 schematically illustrates an overall configuration of a catheter system 1 to which a balloon catheter 10 according to the present embodiment is applied. The balloon catheter 10 according to the present embodiment is devised for improving operability.

The catheter system 1 illustrated in FIG. 1 comprises the balloon catheter (hereinafter, also simply referred to as a "catheter") 10 to and from which liquid is supplied and discharged, a stirring line 60 and a stirring device 65 for stirring the liquid in the catheter 10, and a heating device 75 for heating the liquid in the catheter 10.

The catheter 10 comprises a catheter shaft 20 having a distal end to which a balloon 15 is attached, a supply-discharge line connection portion 30, a stopcock 35, a stirring line connection portion 40, an operation unit 50 for extending the balloon 15, and a housing 55 that accommodates a proximal end portion of the catheter shaft 20. In the illustrated example, the catheter 10 further comprises a supply-discharge line 36 connected to the supply-discharge line connection portion 30, a supply-discharge device 38 connected to the supply-discharge line 36, and a heating electrode 70 electrically connected to the heating device 75 to heat the liquid in the balloon 15.

In the present specification, the terms "distal ends" and "distal end portions" used with respect to the balloon 15, the catheter shaft 20 (including its inner cylinder shaft 22 and outer cylinder shaft 24 to be described later), the operation unit 50, and the housing 55 (including each portion of the housing 55) respectively mean end portions on the side close to the distal end of the catheter 10 extended in a straight line unless otherwise specified. In addition, in the present specification, the terms "proximal ends" and "proximal end portions" used with respect to the balloon 15, the catheter shaft 20, the operation unit 50, and the housing 55 respectively mean end portions opposite to the distal ends of the balloon 15, the catheter shaft 20, the operation unit 50, and the housing 55 unless otherwise specified.

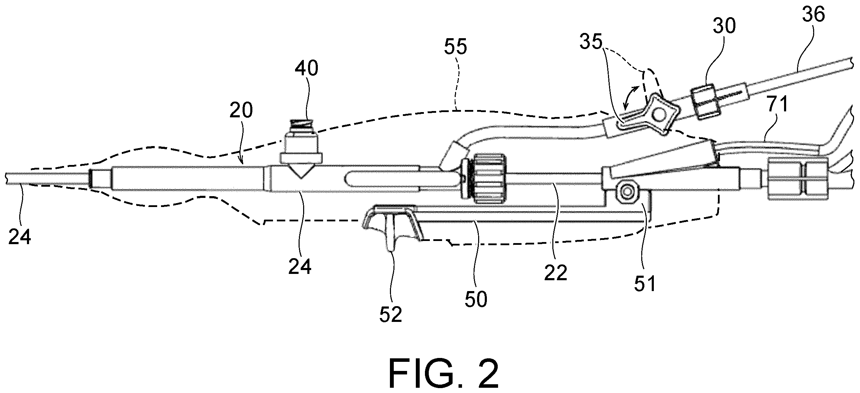

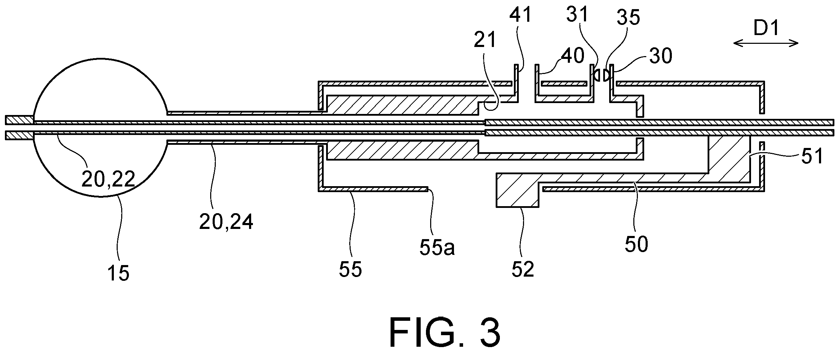

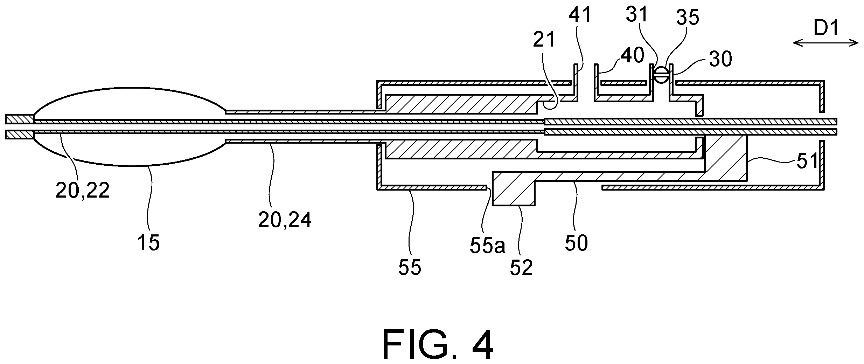

FIG. 2 illustrates a side view of the proximal end portion of the catheter shaft 20. Further, FIGS. 3 and 4 are cross-sectional views schematically illustrating the structure of the catheter shaft 20. FIG. 3 illustrates a cross-section of the catheter shaft 20 with the balloon 15 in a liquid inflatable state in which the balloon 15 is inflatable with liquid, and FIG. 4 illustrates a cross-section of the catheter shaft 20 with the balloon 15 in an extended state.

As illustrated in FIGS. 3 and 4, the catheter shaft 20 is formed with a liquid feeding path 21 for feeding liquid to the balloon 15. The liquid feeding path 21 communicates with the inside of the balloon 15. In the illustrated example, as illustrated in FIGS. 2 to 4, the catheter shaft 20 includes an inner cylinder shaft 22 and an outer cylinder shaft 24. The inner cylinder shaft 22 is inserted into the lumen of the outer cylinder shaft 24. The liquid feeding path 21 is formed between the inner cylinder shaft 22 and the outer cylinder shaft 24. A guide wire (not illustrated) for guiding the catheter shaft 20 in the body of the patient is inserted into the lumen of the inner cylinder shaft 22.

The outer cylinder shaft 24 is not movable with respect to the inside of the housing 55. The inner cylinder shaft 22 is movable relative to the outer cylinder shaft 24 along the longitudinal direction of the catheter shaft 20. In the illustrated example, the inner cylinder shaft 22 is movable between two positions (balloon inflatable position illustrated in FIG. 3 and balloon extension position illustrated in FIG. 4) with reference to the outer cylinder shaft 24. The operation unit 50 for moving the inner cylinder shaft 22 relative to the outer cylinder shaft 24 is fixed to the inner cylinder shaft 22.

The length of the catheter shaft 20 is preferably from 0.5 to 2 m from the viewpoint of allowing the balloon 15 to reach the myocardial tissue. Further, the diameter of the catheter shaft 20 is preferably from 3 to 5 mm from the viewpoint of insertion into a blood vessel. The material of the catheter shaft 20 is preferably a flexible material having excellent antithrombogenicity, and examples thereof include a fluororesin, a polyamide resin, a polyurethane resin, and a polyimide resin, but are not limited thereto.

The internal space of the balloon 15 communicates with the liquid feeding path 21. In the example illustrated in FIGS. 3 and 4, the distal end of the balloon 15 is fixed to the distal end of the inner cylinder shaft 22. Further, the proximal end of the balloon 15 is fixed to the distal end of the outer cylinder shaft 24. Then, by moving the inner cylinder shaft 22 relative to the outer cylinder shaft 24 along the longitudinal direction of the catheter shaft 20, the balloon 15 can be extended in the direction of the relative movement between the inner cylinder shaft 22 and the outer cylinder shaft 24.

The diameter of the balloon 15 is preferably from 20 to 40 mm from the viewpoint of being able to closely contact the site where the arrhythmia occurs. Further, the balloon 15 preferably has a spherical shape. Further, the thickness of the balloon 15 is preferably from 20 to 100 µm. As the material of the balloon 15, a stretchable material excellent in antithrombogenicity is preferable, and a polyurethane-based polymer material is more preferable. Examples of the polyurethane-based polymer material include thermoplastic polyether urethane, polyether polyurethane urea, fluorine polyether urethane urea, polyether polyurethane urea resin, and polyether polyurethane urea amide.

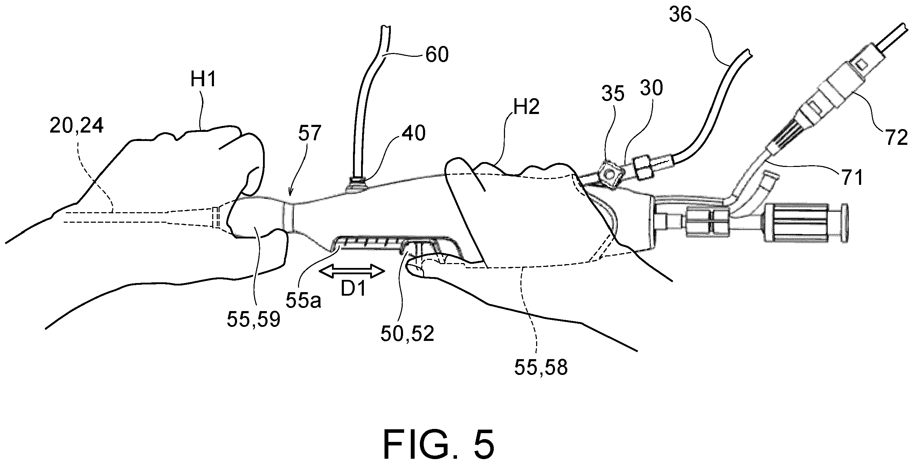

As illustrated in FIGS. 2 to 4, the operation unit 50 is fixed to the inner cylinder shaft 22. In addition, the operation unit 50 is movable relative to the housing 55 in a first direction D1 along the proximal end portion of the catheter shaft 20. Therefore, the operation unit 50 is movable together with the inner cylinder shaft 22 relative to the housing 55 and the outer cylinder shaft 24 in the first direction D1. In the illustrated example, the proximal end portion of the inner cylinder shaft 22 extends from the proximal end of the outer cylinder shaft 24, and a proximal end portion 51 of the operation unit 50 is connected to the proximal end portion of the inner cylinder shaft 22. A distal end portion 52 of the operation unit 50 is exposed to the outside of the housing 55 through an operation unit opening 55a formed in the housing 55, and the operation unit 50 can be operated from the outside of the housing 55. As illustrated in FIG. 5, such an operation unit 50 can be operated with the same hand H2 as the hand H2 gripping the housing 55.

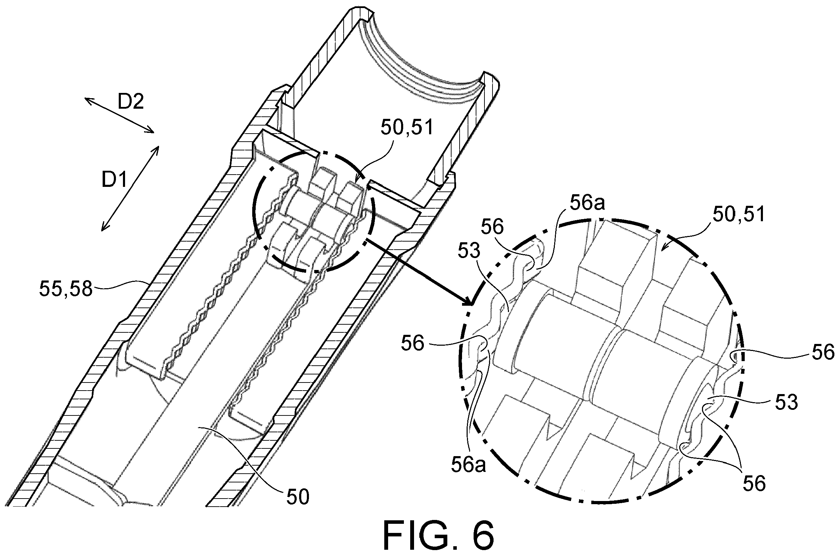

FIG. 6 is a cross-sectional view illustrating the housing 55 and the proximal end portion of the operation unit 50. In the illustrated example, as illustrated in FIG. 6, the proximal end portion 51 of the operation unit 50 is provided with a movement restricting protrusion 53 that protrudes in a second direction D2 intersecting the first direction D1 that is the moving direction of the operation unit 50. The relative movement of the operation unit 50 relative to the housing 55 is restricted by engaging the movement restricting protrusion 53 with any one of a plurality of movement restricting recesses 56 provided along the first direction D1 on the inner wall of the housing 55. This may maintain the balloon 15 at a desired length along the longitudinal direction of the catheter shaft 20.

The engagement between the movement restricting protrusion 53 and the movement restricting recess 56 can be released by the proximal end portion 51 of the operation unit 50 being elastically deformed in the second direction D2. In the illustrated example, since each movement restricting recess 56 is delimited by inclined surfaces of the side wall portions 56b facing one another in the first direction D1, when a force in the first direction D1 is applied to the operation unit 50 in a state where the movement restricting protrusion 53 and the movement restricting recess 56 are engaged, a force in the second direction D2 is applied from the side wall portion 56a to the movement restricting protrusion 53, and the proximal end portion 51 of the operation unit 50 is elastically deformed in the second direction D2. As a result, the movement restricting protrusion 53 moves in the second direction D2, and the engagement between the movement restricting protrusion 53 and the movement restricting recess 56 is released. As a matter of course, the method for releasing the engagement between the movement restricting protrusion 53 and the movement restricting recess 56 is not limited thereto. The engagement between the movement restricting protrusion 53 and the movement restricting recess 56 may be released, for example, by the housing 55 being elastically deformed in the second direction D2.

The supply-discharge line connection portion 30 is formed in a tubular shape and has an internal space 31 communicating with the liquid feeding path 21 (see FIGS. 3 and 4). The liquid is supplied to and discharged from the liquid feeding path 21 through the internal space 31. In the illustrated example, the supply-discharge line connection portion 30 is connected to the proximal end portion of the outer cylinder shaft 24, and the position thereof with respect to the housing 55 is fixed.

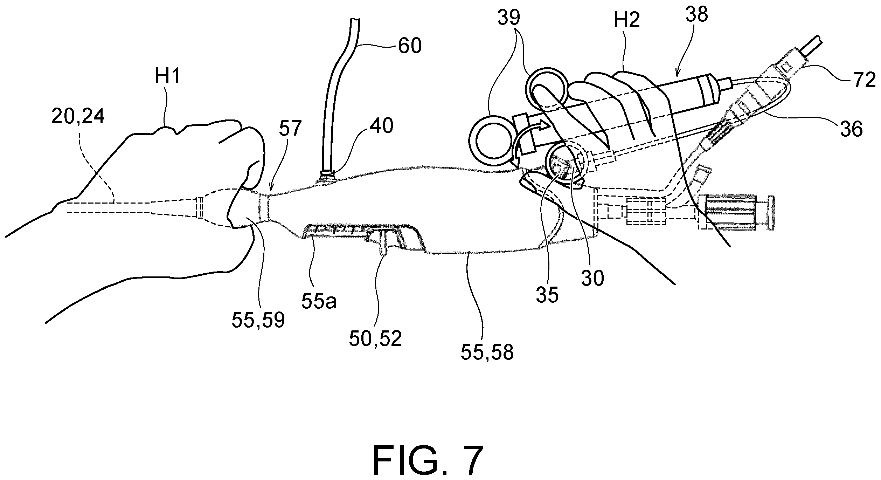

As illustrated in FIG. 1, one end of the supply-discharge line 36 is connected to the supply-discharge device 38, and the other end thereof is connected to the supply-discharge line connection portion 30. The supply-discharge line 36 supplies and discharges liquid to and from the liquid feeding path 21 through the internal space 31 of the supply-discharge line connection portion 30. In the illustrated example, the supply-discharge line 36 includes a soft tube having a length of 200 mm or more. Since the supply-discharge line 36 includes a soft tube having a sufficient length, as illustrated in FIG. 7, it is possible to curve the supply-discharge line 36 in order to operate the stopcock 35 while gripping the supply-discharge device 38 in a state where the supply-discharge device 38 is connected to the catheter shaft 20 via the supply-discharge line 36 and the supply-discharge line connection portion 30. As the material of the supply-discharge line 36, for example, polyvinyl chloride, polyurethane, silicon, polyamide, or the like can be employed.

The supply-discharge device 38 supplies and discharges liquid to and from the supply-discharge line 36. In the illustrated example, the supply-discharge device 38 is a syringe, but is not limited thereto. In the example illustrated in FIG. 1, the supply-discharge device 38 is provided with a ring/rings 39 through which a person who operates the supply-discharge device 38 passes his/her finger/fingers. This makes it easy to operate the stopcock 35 and the like while gripping the supply-discharge device 38. As the liquid supplied and discharged by the supply-discharge device 38, a contrast medium or a contrast medium diluted with physiological saline is preferable so that the balloon 15 inflated with the liquid can be confirmed by an X-ray fluoroscopic image.

The supply-discharge line 36 and the supply-discharge device 38 may be included in the catheter system 1 and may not be included in the balloon catheter 10.

The stopcock 35 is provided in the supply-discharge line connection portion 30. More specifically, a part of the stopcock 35 is arranged in the internal space 31 of the supply-discharge line connection portion 30, and the internal space 31 of the supply-discharge line connection portion 30 can be closed by rotating the stopcock 35 relative to the supply-discharge line connection portion 30 and arranging the stopcock 35 in a predetermined direction. In the illustrated example, the stopcock 35 is rotatable between a closed position indicated by a solid line in FIG. 2 and an open position indicated by a broken line in FIG. 2. When the stopcock 35 is disposed at the closed position, the internal space 31 of the supply-discharge line connection portion 30 is closed, and the communication between the supply-discharge line 36 and the liquid feeding path 21 is blocked. When the stopcock 35 is disposed at the open position, the internal space 31 of the supply-discharge line connection portion 30 is opened, and the supply-discharge line 36 and the liquid feeding path 21 communicate with each other.

As described above, the position of the supply-discharge line connection portion 30 with respect to the housing 55 is fixed. This improves the operability of the stopcock 35. Specifically, first, as illustrated in FIG. 7, the catheter shaft 20 and the supply-discharge line connection portion 30 can be simultaneously retained by retaining the housing 55. In other words, the supply-discharge line connection portion 30 can be retained by a hand holding the catheter shaft 20. Therefore, it is possible to retain the catheter shaft 20 and the supply-discharge line connection portion 30 by gripping the housing 55 with one hand and operate the stopcock 35 with the other hand. That is, it is possible that a person who operates the catheter shaft 20 operates the stopcock 35 while retaining the catheter shaft 20.

The stirring line connection portion 40 is formed in a tubular shape and has an internal space 41 communicating with the liquid feeding path 21 (see FIGS. 3 and 4). The stirring line connection portion 40 is connected with a stirring line 60 that sucks and discharges liquid from and into the liquid feeding path 21 through the internal space 41 of the stirring line connection portion 40. When the stirring line 60 is connected and the liquid is sucked and discharged from and into the liquid feeding path 21, the liquid in the balloon 15 vibrates and is stirred. By stirring the liquid in the balloon 15, the surface temperature of the balloon 15 can be made uniform, and the diseased part can be uniformly cauterized.

Here, the stirring line 60 is connected to the stirring device 65 to suck and discharge the liquid from and into the liquid feeding path 21. The stirring device 65 periodically sucks and discharges the liquid from and into the internal space of the stirring line 60. The stirring device 65 is preferably a device that repeats suction and discharge of liquid 1 to 5 times per 1 second, and preferably includes a pump selected from the group consisting of a roller pump, a diaphragm pump, a bellows pump, a vane pump, a centrifugal pump, and a pump including a combination of a piston and a cylinder.

The stirring line connection portion 40 is connected to the proximal end portion of the outer cylinder shaft 24. In the illustrated example, the stirring line connection portion 40 is fixed to the housing 55. Accordingly, by holding the housing 55, the stirring line connection portion 40 and the stirring line 60 can be held together with the catheter shaft 20.

In the illustrated example, as illustrated in FIG. 2, the stirring line connection portion 40 is provided closer to the distal end portion of the catheter shaft 20 than the supply-discharge line connection portion 30. In other words, as illustrated in FIGS. 3 and 4, the connection portion between the internal space 41 of the stirring line connection portion 40 and the liquid feeding path 21 is located closer to the distal end portion of the catheter shaft 20 than the connection portion between the internal space 31 of the supply-discharge line connection portion 30 and the liquid feeding path 21. As a result, it is possible to control stirring of the liquid and supply and discharge of the liquid independently. Specifically, even during supply of the liquid from the supply-discharge line 36 to the liquid feeding path 21 and discharge of the liquid from the liquid feeding path 21 to the supply-discharge line 36, the liquid can be sucked and discharged from and into the liquid feeding path 21 through the internal space 41 of the stirring line connection portion 40 to stir the liquid in the balloon 15. As a result, even if the liquid is supplied and discharged to and from the liquid feeding path 21 during the cauterization of the diseased part, the surface temperature of the balloon 15 can be uniformly maintained, and the occurrence of uneven cauterization in the diseased part can be prevented. Then, the therapeutic effect can be improved, and the burden on the patient can be reduced.

The stirring line connection portion 40 is not provided with the stopcock 35. In the illustrated example, the stirring line connection portion 40 causes the stirring line 60 and the liquid feeding path 21 to always communicate with each other at least while the stirring line 60 is connected.

As illustrated in FIG. 1, the heating electrode 70 is fixed to the outer peripheral surface of the inner cylinder shaft 22 inside the balloon 15. The distal end of the lead wire 71 is connected to the heating electrode 70. The other end portion of the lead wire 71 is provided with a connector 72 for electrically connecting the lead wire 71 to the heating device 75. Here, the heating device 75 applies electrical energy to the heating electrode 70 via the lead wire 71 to heat the liquid around the heating electrode 70. As the heating device 75, a high frequency generation device capable of generating a high frequency current of 100 Hz or more can be employed, but the present invention is not limited thereto.

In the illustrated example, the lead wire 71 is inserted into the liquid feeding path 21, but the present invention is not limited thereto.

As illustrated in FIG. 1, the housing 55 includes a narrow portion 57 narrowed in a direction intersecting the first direction D1 in which the proximal end portion of the catheter shaft 20 extends, a first gripping portion 58 connected to the narrow portion 57 from one side (proximal end side) in the first direction D1, and a second gripping portion 59 connected to the narrow portion 57 from the other side (distal end side) in the first direction D1. Since the narrow portion 57 is formed in the housing 55, both the first gripping portion 58 and the second gripping portion 59 can be easily gripped. In addition, it is possible to prevent the housing 55 from being displaced in the first direction D1 from the hand of a person who grips the housing 55 (person who operates the catheter 10) while the housing 55 is gripped. As a result, it is possible to prevent the catheter shaft 20 and the balloon 15 from unintentionally moving along the longitudinal direction of the catheter shaft 20 while the housing 55 is gripped. As a result, it is possible to prevent the catheter shaft 20 and the balloon 15 from being unintentionally separated from the diseased part during cauterization of the diseased part.

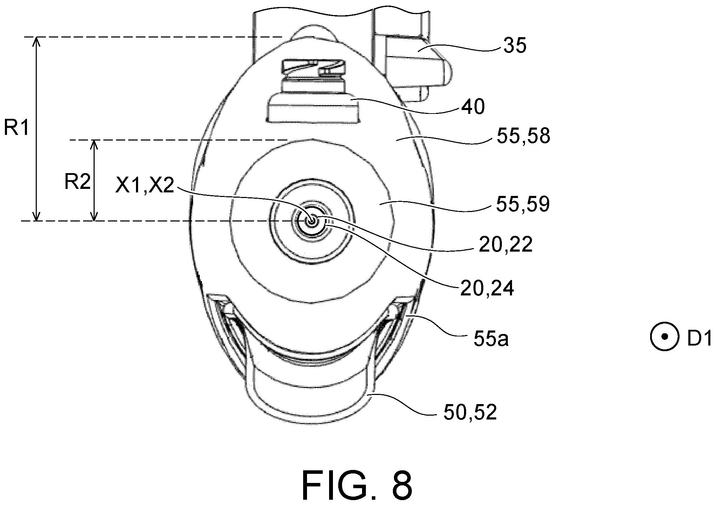

FIG. 8 illustrates a view of the housing 55 as viewed from its distal end side. In FIG. 8, an arrow extending along a direction perpendicular to the paper surface of the drawing is represented by a symbol of a circled dot.

In the illustrated example, as illustrated in FIG. 8, a maximum radius (hereinafter, also referred to as "maximum outer radius") R1 of the circumscribed circle of the first gripping portion 58 about an axis X1 extending along the first direction D1 is larger than a maximum radius (maximum outer radius) R2 of the circumscribed circle of the second gripping portion 59 about an axis X2 extending along the first direction D1. The first gripping portion 58 is provided with the operation unit 50 for extending the balloon 15. As a result, the operation method of the catheter 10 can be visually transmitted. That is, when operating the operation unit 50, it is necessary to apply a relatively large force capable of releasing the engagement between the movement restricting protrusion 53 and the movement restricting recess 56 to the housing 55 and the operation unit 50. Therefore, it is understood that the first gripping portion 58 should be firmly gripped to operate operation unit 50 as illustrated in FIG. 5. On the other hand, in other cases, since it is sufficient to apply a relatively small force to the housing 55, it is understood that it is only necessary to lightly hold the second gripping portion 59 as illustrated in FIG. 7.

Next, an example of a method of operating the catheter system 1 to which the above-described balloon catheter 10 is applied will be described with reference to FIGS. 1 to 5 and 7. Hereinafter, a case where the catheter system 1 is applied to ablation treatment of atrial fibrillation will be described as an example.

First, the distal end of the catheter 10 is inserted into the pulmonary vein. At this time, the inner cylinder shaft 22 is disposed at the balloon extension position illustrated in FIG. 4, and the balloon 15 is extended. No liquid is contained in the balloon 15. Further, the stopcock 35 is disposed at a closed position (position indicated by a solid line in FIG. 2), and the internal space 31 of the supply-discharge line connection portion 30 is closed. Therefore, communication between the supply-discharge line 36 and the liquid feeding path 21 is blocked. In addition, the stirring line 60 is connected to the stirring line connection portion 40 and the heating device 75 is connected to the connector 72, but the stirring device 65 and the heating device 75 are not operated.

After the distal end of the catheter 10 is inserted into the pulmonary vein, as illustrated in FIG. 5, while the second gripping portion 59 of the housing 55 is gripped with one hand H1, the first gripping portion 58 of the housing 55 is firmly gripped with the other hand H2. Then, while gripping the first gripping portion 58 with the other hand H2, the operation unit 50 is moved to the proximal end side of the housing 55 with a finger/fingers of the other hand H2. As a result, the inner cylinder shaft 22 moves to the balloon inflatable position illustrated in FIG. 3.

Next, as illustrated in FIG. 7, while the second gripping portion 59 of the housing 55 is gripped with the one hand H1, the supply-discharge device 38 is gripped with the other hand H2. Then, while gripping the supply-discharge device 38 with the other hand H2, the supply-discharge line 36 is curved, and the stopcock 35 is rotated to the open position (position indicated by the broken line in FIG. 2) with the other hand H2. As a result, the supply-discharge line 36 and the liquid feeding path 21 communicate with each other. Thereafter, the supply-discharge device 38 is operated with the other hand H2 to supply the liquid into the liquid feeding path 21 and the balloon 15.

When the balloon 15 is sufficiently inflated by the supplied liquid, as illustrated in FIG. 7, the supply-discharge line 36 is curved while the supply-discharge device 38 is gripped by the other hand H2, and the stopcock 35 is rotated to the closed position by the other hand H2. As a result, the internal space 31 of the supply-discharge line connection portion 30 is closed, and communication between the supply-discharge line 36 and the liquid feeding path 21 is blocked. Thereafter, the stirring device 65 and the heating device 75 are operated to stir the liquid in the balloon 15 while heating the liquid. As a result, the diseased part is cauterized. During the cauterization, the catheter shaft 20 and/or the second gripping portion 59 of the housing 55 are held with one hand H2 and/or both hands H1 and H2 so that the balloon 15 is not separated from the diseased part, and the catheter shaft 20 is retained against the diseased part.

When it is determined that the balloon 15 has insufficient liquid or the balloon 15 has excessive liquid during cauterization, the liquid is supplied or discharged while the stirring of the liquid in the balloon 15 by the stirring device 65 is continued. Specifically, as illustrated in FIG. 7, the supply-discharge device 38 is gripped with the other hand H2, the supply-discharge line 36 is curved while the supply-discharge device 38 is gripped, and the stopcock 35 is rotated to the open position with the other hand H2. As a result, the liquid feeding path 21 and the supply-discharge line 36 communicate with each other. Then, the supply-discharge device 38 is operated with the other hand H2 to supply and discharge the liquid to and from the liquid feeding path 21 and the balloon 15. During this time, since the liquid in the balloon 15 continues to be stirred by the stirring device 65, the temperature of the surface of the balloon 15 is maintained uniform. Then, when the amount of the liquid in the balloon 15 reaches an appropriate amount, as illustrated in FIG. 7, the supply-discharge line 36 is curved while the supply-discharge device 38 is gripped with the other hand H2, and the stopcock 35 is rotated to the closed position with the other hand H2. As a result, communication between the liquid feeding path 21 and the supply-discharge line 36 is blocked.

When the cauterization is completed, the stirring device 65 and the heating device 75 are stopped. Then, the liquid is discharged from the balloon 15 and the liquid feeding path 21. Specifically, as illustrated in FIG. 7, while the second gripping portion 59 of the housing 55 is gripped with the one hand H1, the supply-discharge device 38 is gripped with the other hand H2, the supply-discharge line 36 is curved while the supply-discharge device 38 is gripped, and the stopcock 35 is rotated to the open position with the other hand H2. As a result, the liquid feeding path 21 and the supply-discharge line 36 communicate with each other. Then, the supply-discharge device 38 is operated with the other hand H2 to discharge the liquid from the balloon 15 and the liquid feeding path 21. When the liquid in the balloon 15 is sufficiently discharged, the supply-discharge line 36 is curved while holding the supply-discharge device 38 with the other hand H2, and the stopcock 35 is rotated to the closed position with the other hand H2. As a result, communication between the liquid feeding path 21 and the supply-discharge line 36 is blocked.

Next, the balloon 15 is extended while gripping the second gripping portion 59 of the housing 55 with the one hand H1. Specifically, as illustrated in FIG. 5, the other hand H2 firmly grips the first gripping portion 58 of the housing 55, and a finger/ fingers of the other hand H2 move the operation unit 50 to the distal end side of the housing 55. As a result, the inner cylinder shaft 22 moves to the balloon extension position illustrated in FIG. 4. Once the balloon 15 is sufficiently extended, the catheter 10 is withdrawn from the pulmonary vein.

As described above, according to the present embodiment, the balloon catheter 10 comprises the housing 55, the catheter shaft 20 having the proximal end portion disposed in the housing 55 and forming the liquid feeding path 21 communicating with the inside of the balloon 15 attached to the distal end portion of the catheter shaft, the supply-discharge line connection portion 30 which has the internal space 31 communicating with the liquid feeding path 21, and to which the supply-discharge line 36 that supplies and discharges the liquid to and from the liquid feeding path 21 through the internal space 31 can be connected, and the stopcock 35 capable of closing the internal space 31 of the supply-discharge line connection portion 30. The position of the supply-discharge line connection portion 30 with respect to the housing 55 is fixed. According to such a balloon catheter 10, the operability of the stopcock 35 is improved.

Further, the balloon catheter 10 according to the present embodiment further comprises the supply-discharge line 36 connected to the supply-discharge line connection portion 30 and the supply-discharge device 38 that is connected to the supply-discharge line 36 to supply and discharge the liquid to and from the supply-discharge line 36. The supply-discharge line 36 includes a soft tube having a length of 200 mm or more. According to such a balloon catheter 10, the stopcock 35 can be operated while gripping the supply-discharge device 38 in a state where the supply-discharge device 38 is connected to the catheter shaft 20 via the supply-discharge line 36 and the supply-discharge line connection portion 30.

The balloon catheter 10 according to the present embodiment further comprises the stirring line connection portion 40 which has the internal space 41 communicating with the liquid feeding path 21, and to which the stirring line 60 that sucks and discharges the liquid from and into the liquid feeding path 21 through the internal space 41 to stir the liquid in the balloon 15 can be connected. The connection portion between the internal space 41 of the stirring line connection portion 40 and the liquid feeding path 21 is located closer to the distal end portion of the catheter shaft than the connection portion between the internal space 31 of the supply-discharge line connection portion 30 and the liquid feeding path 21. According to such a balloon catheter 10, even during supply of the liquid from the supply-discharge line 36 to the liquid feeding path 21 and discharge of the liquid from the liquid feeding path 21 to the supply-discharge line 36, the liquid can be sucked and discharged from and into the liquid feeding path 21 through the internal space 41 of the stirring line connection portion 40 to stir the liquid in the balloon 15. As a result, even if the liquid is supplied and discharged to and from the liquid feeding path 21 during the cauterization of the diseased part, the surface temperature of the balloon 15 can be uniformly maintained, and the occurrence of uneven cauterization in the diseased part can be prevented. Then, the therapeutic effect can be improved, and the burden on the patient can be reduced.

In addition, in the balloon catheter 10 according to the present embodiment, the stirring line connection portion 40 is fixed to the housing 55. Accordingly, by holding the housing 55, the stirring line connection portion 40 and the stirring line 60 can be held together with the catheter shaft 20.

In addition, in the balloon catheter 10 according to the present embodiment, the catheter shaft 20 includes the inner cylinder shaft 22 to which the distal end of the balloon 15 is fixed, and the outer cylinder shaft 24 to which the proximal end of the balloon 15 is fixed, the outer cylinder shaft 24 having a lumen into which the inner cylinder shaft 22 is inserted to form the liquid feeding path 21 between the outer cylinder shaft 24 and the inner cylinder shaft 22. The housing 55 is provided with the operation unit 50 that moves the inner cylinder shaft 22 relative to the outer cylinder shaft 24 along the first direction D1 in which the proximal end portion of the catheter shaft 20 extends in order to extend the balloon 15. According to such a balloon catheter 10, the operation unit 50 can be operated with the same hand H2 as the hand H2 gripping the housing 55.

In addition, in the balloon catheter 10 according to the present embodiment, the housing 55 includes the narrow portion 57 narrowed in the direction intersecting the first direction D1 in which the proximal end portion of the catheter shaft 20 extends, the first gripping portion 58 connected to the narrow portion 57 from one side in the first direction D1, and the second gripping portion 59 connected to the narrow portion 57 from the other side in the first direction D1. According to such a balloon catheter 10, it is possible to prevent the housing 55 from being displaced in the first direction D1 from the hand of a person who grips the housing 55 (person who operates the catheter 10) while the housing 55 is gripped. As a result, it is possible to prevent the catheter shaft 20 and the balloon 15 from unintentionally moving along the longitudinal direction of the catheter shaft 20 while the housing 55 is gripped. As a result, it is possible to prevent the catheter shaft 20 and the balloon 15 from being unintentionally separated from the diseased part during cauterization of the diseased part.

In addition, in the balloon catheter 10 according to the present embodiment, the catheter shaft 20 includes the inner cylinder shaft 22 to which the distal end of the balloon 15 is fixed, and the outer cylinder shaft 24 to which the proximal end of the balloon 15 is fixed, the outer cylinder shaft 24 having a lumen into which the inner cylinder shaft 22 is inserted to form the liquid feeding path 21 between the outer cylinder shaft 24 and the inner cylinder shaft 22. The maximum outer radius R1 of the first gripping portion 58 about the axis X1 extending along the first direction D1 is larger than the maximum outer radius R2 of the second gripping portion 59 about the axis X2 extending along the first direction D1, and the first gripping portion 58 is provided with the operation unit 50 that moves the inner cylinder shaft 22 relative to the outer cylinder shaft 24 along the first direction D1 to stretch the balloon 15. According to such a balloon catheter 10, the operation method of the catheter 10 can be visually transmitted. That is, it is understood that when operating the operation unit 50, the first gripping portion 58 should be firmly gripped to operate the operation unit 50 as illustrated in FIG. 5. On the other hand, in other cases, it is understood that the second gripping portion 59 may be lightly held as illustrated in FIG. 7.

Alternatively, the balloon catheter 10 according to the present embodiment comprises the housing 55 and the catheter shaft 20 having the proximal end portion disposed in the housing 55 and forming the liquid feeding path 21 communicating with the inside of the balloon 15 attached to the distal end portion of the catheter shaft 20. The housing 55 includes the narrow portion 57 narrowed in the direction intersecting the first direction D1 in which the proximal end portion of the catheter shaft 20 extends, the first gripping portion 58 connected to the narrow portion 57 from one side in the first direction D1, and the second gripping portion 59 connected to the narrow portion 57 from the other side in the first direction D1. According to such a balloon catheter 10, it is possible to prevent the housing 55 from being displaced in the first direction D1 from the hand of a person who grips the housing 55 (person who operates the catheter 10) while the housing 55 is gripped. As a result, it is possible to prevent the catheter shaft 20 and the balloon 15 from unintentionally moving along the longitudinal direction of the catheter shaft 20 while the housing 55 is gripped. As a result, it is possible to prevent the catheter shaft 20 and the balloon 15 from being unintentionally separated from the diseased part during cauterization of the diseased part.

Alternatively, the balloon catheter 10 according to the present embodiment comprises the housing 55, the catheter shaft 20 having the proximal end portion disposed in the housing 55 and forming the liquid feeding path 21 communicating with the inside of the balloon 15 attached to the distal end portion of the catheter shaft 20, the supply-discharge line connection portion 30 which has the internal space 31 communicating with the liquid feeding path 21, and to which the supply-discharge line 36 that supplies and discharges the liquid to and from the liquid feeding path 21 through the internal space 31 can be connected, and the stirring line connection portion 40 which has the internal space 41 communicating with the liquid feeding path 21, and to which the stirring line 60 that sucks and discharges the liquid from and into the liquid feeding path 21 through the internal space 41 to stir the liquid in the balloon 15 can be connected. The connection portion between the internal space 41 of the stirring line connection portion 40 and the liquid feeding path 21 is located closer to the distal end portion of the catheter shaft 20 than the connection portion between the internal space 31 of the supply-discharge line connection portion 30 and the liquid feeding path 21. According to such a balloon catheter 10, even during supply of the liquid from the supply-discharge line 36 to the liquid feeding path 21 and discharge of the liquid from the liquid feeding path 21 to the supply-discharge line 36, the liquid can be sucked and discharged from and into the liquid feeding path 21 through the internal space 41 of the stirring line connection portion 40 to stir the liquid in the balloon 15. As a result, even if the liquid is supplied and discharged to and from the liquid feeding path 21 during the cauterization of the diseased part, the surface temperature of the balloon 15 can be uniformly maintained, and the occurrence of uneven cauterization in the diseased part can be prevented. Then, the therapeutic effect can be improved, and the burden on the patient can be reduced.

The above-described embodiment and modifications thereof are included in the scope and gist of the invention, and are included in the invention described in the claims and the equivalent scope thereof. In addition, as a matter of course, it is also possible to partially appropriately combine the above-described embodiment and modifications thereof within the scope of the gist of the present invention.

### REFERENCE SIGNS LIST

- 1: catheter system
- 1, 10: balloon catheter
- 15: balloon
- 20: catheter shaft
- 22: inner cylinder shaft
- 24: outer cylinder shaft
- 30: supply-discharge line connection portion
- 31: internal space of supply-discharge line connection portion
- 35: stopcock
- 40: stirring line connection portion
- 41: internal space of stirring line connection portion
- 50: operation unit
- 55: housing
- 57: narrow portion
- 58: first gripping portion
- 59: second gripping portion
- 60: stirring line
- 65: stirring device
- 70: heating electrode
- 71: lead wire
- 75: heating device

## Claims

1. A balloon catheter comprising:
a housing;
a catheter shaft having a proximal end portion disposed in the housing and forming a liquid feeding path communicating with an inside of a balloon attached to a distal end portion of the catheter shaft;
a supply-discharge line connection portion which has an internal space communicating with the liquid feeding path, and to which a supply-discharge line that supplies and discharges liquid to and from the liquid feeding path through the internal space can be connected; and
a stopcock capable of closing the internal space of the supply-discharge line connection portion,
wherein a position of the supply-discharge line connection portion with respect to the housing is fixed.

2. The balloon catheter according to claim 1, comprising:
the supply-discharge line connected to the supply-discharge line connection portion; and
a supply-discharge device that is connected to the supply-discharge line to supply and discharge liquid to and from the supply-discharge line,
wherein the supply-discharge line includes a soft tube having a length of 200 mm or more.

3. The balloon catheter according to claim 1 or 2, comprising
a stirring line connection portion which has an internal space communicating with the liquid feeding path, and to which a stirring line that sucks and discharges liquid from and into the liquid feeding path through the internal space of the stirring line connection portion to stir liquid in the balloon can be connected,
wherein a connection portion between the internal space of the stirring line connection portion and the liquid feeding path is located closer to the distal end portion of the catheter shaft than a connection portion between the internal space of the supply-discharge line connection portion and the liquid feeding path.

4. The balloon catheter according to any one of claims 1 to 3,
wherein the catheter shaft includes an inner cylinder shaft to which a distal end of the balloon is fixed and an outer cylinder shaft to which a proximal end of the balloon is fixed, the outer cylinder shaft having a lumen into which the inner cylinder shaft is inserted to form the liquid feeding path between the outer cylinder shaft and the inner cylinder shaft, and
the housing is provided with an operation unit that extends the balloon by moving the inner cylinder shaft relative to the outer cylinder shaft along a first direction in which the proximal end portion of the catheter shaft extends.

5. The balloon catheter according to any one of claims 1 to 4, wherein the housing includes a narrow portion narrowed in a direction intersecting a first direction in which the proximal end portion of the catheter shaft extends, a first gripping portion connected to the narrow portion from one side in the first direction, and a second gripping portion connected to the narrow portion from the other side in the first direction.

6. The balloon catheter according to claim 5,
wherein the catheter shaft includes an inner cylinder shaft to which a distal end of the balloon is fixed and an outer cylinder shaft to which a proximal end of the balloon is fixed, the outer cylinder shaft having a lumen into which the inner cylinder shaft is inserted to form the liquid feeding path between the outer cylinder shaft and the inner cylinder shaft,
a maximum outer radius of the first gripping portion about an axis extending along the first direction is larger than a maximum outer radius of the second gripping portion about an axis extending along the first direction, and
the first gripping portion is provided with an operation unit that moves the inner cylinder shaft relative to the outer cylinder shaft along the first direction to extend the balloon.

7. A balloon catheter comprising:
a housing; and
a catheter shaft having a proximal end portion disposed in the housing and forming a liquid feeding path communicating with an inside of a balloon attached to a distal end portion of the catheter shaft,
wherein the housing includes a narrow portion narrowed in a direction intersecting a first direction in which the proximal end portion of the catheter shaft extends, a first gripping portion connected to the narrow portion from one side in the first direction, and a second gripping portion connected to the narrow portion from the other side in the first direction.

8. A balloon catheter comprising:
a housing;
a catheter shaft having a proximal end portion disposed in the housing and forming a liquid feeding path communicating with an inside of a balloon attached to a distal end portion of the catheter shaft;
a supply-discharge line connection portion which has an internal space communicating with the liquid feeding path, and to which a supply-discharge line that supplies and discharges liquid to and from the liquid feeding path through the internal space of the supply-discharge line connection portion can be connected; and
a stirring line connection portion which has an internal space communicating with the liquid feeding path, and to which a stirring line that sucks and discharges liquid from and into the liquid feeding path through the internal space of the stirring line connection portion to stir liquid in the balloon can be connected,
wherein a connection portion between the internal space of the stirring line connection portion and the liquid feeding path is located closer to the distal end portion of the catheter than a connection portion between the internal space of the supply-discharge line connection portion and the liquid feeding path.
